(19) Europäisches Patentamt
European Patent Office
Office européen des brevets

(11) Publication number: 0 198 207 B1

(12) # EUROPEAN PATENT SPECIFICATION

(45) Date of publication of the patent specification: 20.12.89

(51) Int. Cl.⁴: C12Q 1/68, C07H 21/00

(21) Application number: 86103106.0

(22) Date of filing: 08.03.86

(54) **Specific iodination of nucleic acid.**

(30) Priority: 21.03.85 US 714306

(43) Date of publication of application: 22.10.86 Bulletin 86/43

(45) Publication of the grant of the patent: 20.12.89 Bulletin 89/51

(84) Designated Contracting States: AT BE CH DE FR GB IT LI NL SE

(56) References cited:
EP-A- 0 097 373
WO-A-84/03285
US-A- 4 310 675
US-A- 4 397 779

CHEMICAL ABSTRACTS, vol. 88, no. 17, 24th April 1978, page 215, abstract no. 117161m, Columbus, Ohio, US; G.M. TENER et al.: "Synthesis of iodine-125-labeled N-3-(4-hydroxyphenyl)propionyl aminoacyl transfer ribonucleic acids", BIOCHEMISTRY 1978, 17(4), 741-5a pig papillary muscle" Seite 34, Spalte 2, Zusammenfassung-Nr. 400j 000

(73) Proprietor: MOLECULAR DIAGNOSTICS, INC., 400 Morgan Lane, West Haven, CT 06516(US)

(72) Inventor: Dattagupta, Nanibhushan, 470 Prospect Street, New Haven, Ct 06511(US)
Inventor: Knowles, William, Dr., 101 Sleeping Giant Drive, Hamden Connecticut(US)

(74) Representative: Adrian, Albert, Dr. et al, c/o BAYER AG Konzernverwaltung RP Patentabteilung, D-5090 Leverkusen 1, Bayerwerk(DE)

EP 0 198 207 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

BACKGROUND OF THE INVENTION

This application relates to novel process for producing iodinated nucleic acid probes.

Synthetic oligonucleotides have been widely used for different purposes in molecular biology. They have been used to introduce and detect specific mutation, to isolate specific genes, and for many other biological purposes (Gillam S., Jahnake, P., Astell, C., Phillips, S., Hutchinson, C.A., and Smith, M., "Defined Transversion Mutations At A Specific Position In DNA Using Synthetic Oligodeoxyribonucleotides As Mutagens." Nucleic Acids Res., 6, 2973–2985, (1979); Gillam, S., and Smith, M., "Site-Specific Mutagenesis Using Synthetic Oligodeoxynucleotide Primers: 1. Optimum Conditions And Minimum Oligodexyribonucleotide Length". Gene, 8, 81–97, (1979); Gillam, S., and Smith, M., "Site-Specific Mutagenesis Using Oligodeoxy-nucleotide Primers: II. In Vitro Selection Of Mutant DNA." Gene, 8, 99-106, (1979); Hutchinson, C.A., Phillips, S., Edgell, M.H., Gillam, S., Jahnake, P., and Smith, M. "Mutagenesis At A Specific Position In An DNA Sequence", J. Biol. Chem., 253, 6551–6560, (1978); Suggs, S.V., Wallace, R.B., Hirose, T., Kawashima, E.H., and Itakura, K., "Use Of Synthetic Oligonucleo-tides As Hybridization Probes: Isolation Of Cloned cDNA Sequences For Human $B_1$-microglobulin", Proc. Natl. Acad. Sci. USA, 78, 6613–6617, (1981); Razin, A., Hirose, T., Itakura, K., and Riggs, A.D., "Efficient Correction Of A Mutation By Use Of Chemically Synthesized DNA", Porc. Natl. Acad. Sci. USA, 75, 4268–4270, (1978); Reyes A.A., Johnson, M.J., Schold, M., Ito, H., Ike, Y., Morin, C., Itakura, K., and Wallace, R.B., "Identification of an H–$2K^b$-Related Molecule By Molecular Cloning", Immunogenetics, 14, 383–392, (1981); Reyes, A.A., Schold, M., Itakura, K., and Wallace, R.B., "Isolation Of A cDNA Clone For The Murine Transplanation Antigen H-$2K^b$", Proc. Natl. Acad. Sci. USA, 79, 3270-3274, (1982); Wallace, R.B., Johnson, P.F., Tanaka, S., Schold, M., Itakura, K., and Abelson, J., "Directed Deletion Of A Yeast Transfer RNA Intervening Sequence", Science, 209, 1396-1400, (1980); Wallace, R.B., Schold, M., Johnson, M.J., Dembek, P., and Itakura, K., "Oligonucleotide Directed Mutagenesis Of The Human β-Globin Gene: A General Method For Producing Specific Point Mutations In Cloned DNA", Nucleic Acids Res., 9, 3647-3656, (1981); Wasylyk, B., Derbyshire, R., Guy, A., Molko, D., Roget, A., Teoule, R., And Chambon, P., "Specific In Vitro Transcription Of Conalbumin Gene Is Drastically Decreased By Single Point Mutation In T-A-T-A Box Homology Sequence." Proc. Natl.Acad. Sci. USA, 77, 7024-7028, (1981)).

The detection of specific mutation sites using an oligonucleotide has been used for diagnostic purposes. Conner, B.J. Reyes, A.A., Morin, C., Itakura, K., Teplitz, R.L. and Wallace, R.B., "Detection Of Sickle $\beta^s$-Globin Allele By Hybridization With Synthetic Oligonucleotides", Proc. Natl. Acad. Sci. USA, 80 278-282, (1983), have shown that a specific 19-mer oligonucleotide probe can distinguish between DNA extracted from a sickle cell patient and a normal person. All these methods require that the oligonucleotide be labeled with some detectable radioactive isotope. Since the level of hybridization and the amount of the gene present in a few ml sample of blood or a few grams of tissue is low, nonradioactive detection of the oligonucleotide hybridized to the test is not yet practical. The radioactive methods which are sensitive enough to detect the small amount of DNA in such samples create numerous potential hazards. To alleviate those problems one must use a fast and efficient labeling process. The process should be such that the labeled material can be purified easily and the labeled material should be stable enough to go through the analytical testing procedures essential for the validity of the results.

To date the most efficient method of labeling of an oligonucleotide probe has been the use of polynucleotide kinase to introduce a radioactive phosphorous isotope at the 5' terminus of the nucleic acid. This procedure involves the use of an enzyme which is not stable enough to give reproducible results over a period of time; also it is limited to the use of a phosphorous isotope which has a very short half-life. The use of other enzymes for labeling purposes has similar disadvantages. For example, if one uses terminal deoxynucleotidyl transferase (TdT) to label an oligonucleotide, one cannot control the extent of labeling without the use of dideoxynucleoside triphosphate. After the labeling, the purification of the labeled material also will create some problem where there is an enzyme present in the system.

One approach to this problem involves chemically-labeling the end of an oligonucleotide or a nucleic acid probe by oxidative coupling of nucleoside phosphates or nucleosides and also by reaction with Bolton-Hunter reagent (Bolton, A.E., and Hunter, W.M., Biochem. J., 133, 529, (1973)). We have shown that properly modified oligonucleotide can be iodinated after reaction with Bolton--Hunter reagent or properly reactive nucleosides or nucleoside phosphates. The reaction with the Bolton-Hunter reagent although efficient and fast, is somewhat difficult to perform on a routine basis because of the lability of the starting material. The labeled oligonucleotide has to be purified from the hydrolyzed Bolton-Hunter products which can be conducted by running gel electrophoresis. This procedure is time-consuming and requires handling large amounts of unincorporated isotope.

Several radioactive isotopes of iodine atom can be used to label nucleic acids for specific purposes (Commerford, S.L., Biochemistry, 10, 1993, (1971). Since iodine has isotopes with different half-lives a suitable iodination reagent or a suitable iodinatable nucleic acid will be useful for different purposes. Iodination using iodine monochloride requires that the nucleic acid should be soluble in an organic solvent and that is difficult because in organic solvents most of the nucleic acids will degrade and the solubility is

very low. Iodination using N-iodosuccinimide produces very low yield of the product. Use of thallic chloride causes some degradation of the nucleic acids. All the iodination processes iodinate nucleic acid bases which have some effect on the hybridization property of the nucleic acid probe. The present method is a method of iodination at a specific site and does not alter the hybridization properties of the nucleic acid.

## SUMMARY OF THE INVENTION

It is accordingly an object of the invention to provide nucleic acid probes which are radiolabeled in a way which avoids the aforementioned disadvantages.

These and other objects and advantages are realized in accordance with the present invention pursuant to which there is provided a method of iodinating a nucleic acid by prelabeling the nucleic acid with a cold (non-radioactive) iodinatable residue. The nucleic acid probe which is to be iodinated is first chemically modified to provide a residue which can be catalytically or enzymatically iodinated, leaving the nucleic acid bases intact. Moreover, the present invention also provides a fast and efficient method of separation of the later iodinated material from the starting material and the other precursors, advantageously using a column chromatographic device under high pressure. The nucleic acid probe of the present invention in its iodinated form can be used in nucleic acid hybridization, in particular in nucleic acid hybridization assays.

It is known that lactoperoxidase or chloramine T can be used to iodinate certain aromatic residues (e.g., tyrosine, hydroxyphenyl residue). The present invention makes use of such chemistry. The present invention is a two-step reaction. The nucleic acid probe to be iodinated is first reacted with a reagent which will provide a residue which can be catalytically iodinated leaving ther nucleic acid structurally and functionally intact. The probe comprises a nucleic acid, e.g., an oligonucleotide, fragment covalently linked to an iodinatable residue, e.g., an aryl radical, e.g., phenyl, naphthyl, anthracyl, and the like, optionally substituted with radicals which do not interfere with iodination, e.g., halo, OH, $NH_2$, CN, alkyl and the like. Preferred aryl radicals for the present invention include hydroxyphenyl radicals, indole radicals and imidazole radicals. The linkage of the nucleic acid fragment to the aryl radical can be via a –CON– radical.

The present invention provides a process for producing an iodinated probe of the formula

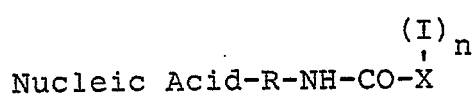

wherein X is an iodinatable residue, R is an aliphatic hydrocarbon radical of up to 11 carbon atoms and n = 1 to 4, which process comprises reacting a nucleic acid having a nucleotide residue containing a primary amino radical with a compound having the formula

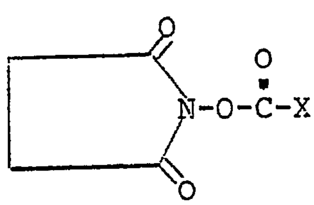

or

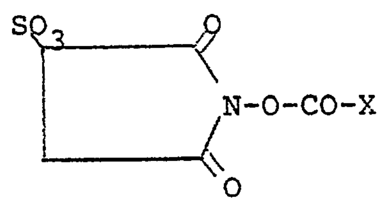

wherein

X is defined above an iodinating the product with an iodinating agent.

In the probe of the above formula

R is an aliphatic hydrocarbon radical of up to 11 carbon atoms, preferably between 3 and 6 carbon atoms and

X is an unsubstituted aryl radical, e.g., phenyl, naphthyl, anthracyl or an aryl radical substituted by a radical which does not interfere with iodination, e.g., halo, OH, $NH_2$, CN and alkyl. A particularly preferred X is

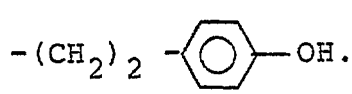

R can be disposed on the sugar or heterocyclic moiety of the nucleic acid.

Purification is done by a fast and efficient method of separation of the iodinated material from the starting material and the other precursors. Described is a method of separation of such iodinated material and their precursors from the starting material and other reactants. The method utilizes a chromatographic device with a hydrophobic bonded phase for a reverse-phase separation. It is known that on a reverse-phase column system one can separate molecules primarily on the basis of their hydrophobic property. In order to utilize that property of a nucleic acid probe one must be able to discriminate the labeled material from the unlabeled material on the basis of their hydrophobic character. The purification uses a high pressure liquid chromatography (HPLC) method of separation of an oligonucleotide from a derivatized oligonucleotide and from an iodine-labeled, modified oligonucleotide. The invention is illustratively described in the following scheme.

The invention involves reacting a nucleic acid having a primary amino radical with

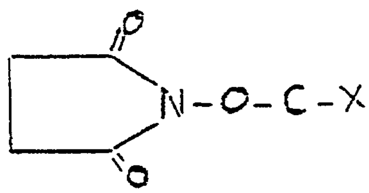

or

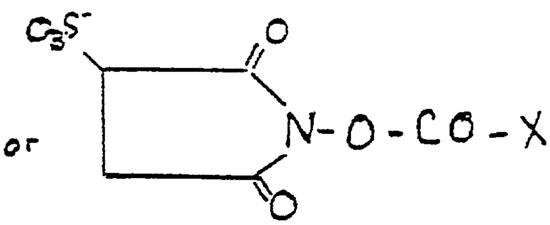

wherein

X is as defined above.

An example of this reaction is described below starting with an amine-containing oligonucleotide:

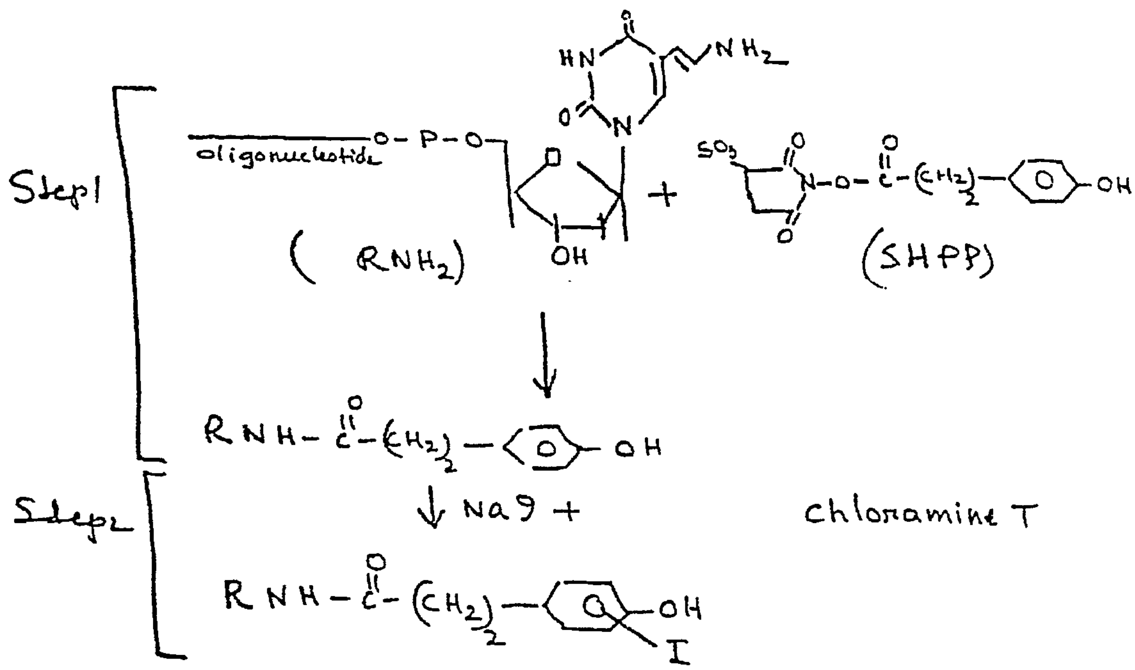


Amine containing nucleotide residue can be an 5-allylaminodU, or 8-or 6-hexylaminodA or A, or a 5-allylamino C or C, oxidatively coupled amino containing A, G, T, C, U. The bond between $-NH_2$ and the iodinatable residue can be N-C, C-C, C-S, S-S, -C-O. In order to create linkages other than -N-C-, the $-NH_2$ residue has to be properly modified with known reagents, e.g., reaction of $RNH_2$ with m-maleimidobenzoyl-N-hydroxysuccinimide ester will provide a third reacting group to form a thioether -C-S-C linkage.

The nucleic acid probe is first modified or prepared in a way such that it will carry a primary amine residue which is reactive with a reagent which carries a catalytically iodinatable molecule such as sulfo-succinimidyl hydroxyphenylpropionate. After covalent attachment of the hydroxyphenyl residue to the oligonucleotide the hydroxyphenyl residue can be iodinated under mild conditions in presence of chloramine T, or lactoperoxidase, or similar reagents.

An iodinatable residue is a radical which can be covalently modified with iodine under specific conditions, such that the rest of the molecule (nucleic acid part) will not change the property. Suitable iodinatable radicals comprise hydroxyaryl, preferably hydroxyphenyl, radicals such as

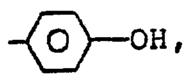

tryptophane, histidine, methionine, cysteine and indole, e.g., cystine. All these iodinatable radicals can be covalently linked to a nucleic acid - amine by known reactions.

Specifically, a nucleic acid with primary aliphatic amine residue (e.g., allylamino U or hexyl amino A) will react with N-hydroxy succinimide ester of a protective amino acid. Such reactions with tyrosine ester will provide

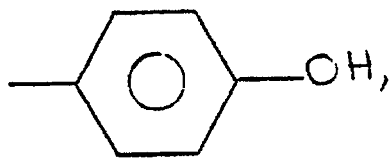

with tryptophane (trp)

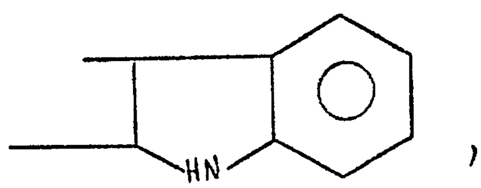

with hydroxy trp

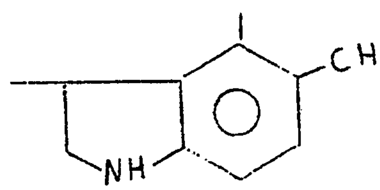

with histidine

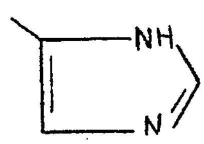

with other amino acids other iodinatable residues can be introduced.

After the reaction of such iodinatable reagents with the oligonucleotide the product has to be purified from the starting reactants in order to get a pure derivatized oligonucleotide for subsequent reactions. The hydroxyphenyl residue labeled nucleic acid probe is then purified on a reverse-phase system and then iodinated in the presence of chloramine T or other catalysts. After iodination in the presence of chloramine T or other catalysts, the major radioactive impurity is iodine. In order to remove iodine from the iodinated nucleic acid and non-iodinated nucleic acid a small reverse-phase column can be used. Routinely this column consists of a wide pore silica (30 nm) coated with a C-18 phase (Commercially available from Brownlee Labs, Inc., Santa Clara, CA. as Aquapore RP-300). The size of the column is 3 cm x 0.46 cm I.D. and has the absorptive capacity to bind a minimum of 5μg oligonucleotide in aqueous solution.

After the major amount of sodium $^{125}$iodide is removed, the separation of the iodinated oligonucleotide from the non-iodinated oligonucleotide can be effected on a simple analytical column using acetonitrile gradient. Once the iodinated material is separated from the non-iodinated material, substantially all oligonucleotides in the fraction containing the iodinated material will be labeled with at least one radioactive isotope. After the iodinated material is separated from the rest of the materials, the sample is tested by hybridization with an unknown sample and a known sample and also by running gel electrophoresis. Although the whole procedure normally involves reaction with an iodinatable residue, separation, iodination, and separation, the whole procedure can be carried out within a couple of hours. Moreover, all intermediate products are stable, allowing a large scale production and storage of the derivatized oligonucleotides which can be labeled with $^{125}$I whenever necessary. The process can be automated in whole or

in part to save time and material, and to improve the purity of the product and the effectiveness of the process, by choosing proper concentration conditions and proper solvent and buffer. Thus, one can use commercially available immobilized chloramine T as a catalyst, so that after the iodination reaction the only impurity will be sodium iodide which can be removed very easily on a simple guard column.

The invention is also illustratively described in the following examples wherein all parts are by weight unless otherwise expressed. Although the example is given with a specific oligonucleotide, similar procedure can be adapted for a ribo oligonucleotide, deoxyribo oligo-nucleotide, a DNA, or RNA probes. The reaction can be carried out with double-stranded DNA, single-stranded DNA, double-stranded RNA, single-stranded RNA, or RNA/DNA hybrid or mixed nucleic acid probes or with a simple di or mono nucleotide or nucleoside phosphates, which can be used as a substrate for different enzymes. All the nucleic acid samples, ribo, dioxy ribo or mixed oligo-or poly-nucleotides can be modified with a primary amine containing nucleotide triphosphate, an enzyme, such as TDT, which will result in a nucleic acid-containing primary $NH_2$ residues. In turn this will react with activated esters of aromatic acids to provide aryl residues on the nucleic acids. An amine-containing nucleoside phosphate will also react similarly to produce iodinatable residue-containing enzyme substrates for nucleic acid modification. As catalysts immobilized or soluble chloramine T, lacto-peroxidase and others can be used.

Example 1

Synthesis of a reactive amine-containing oligonucleotide:

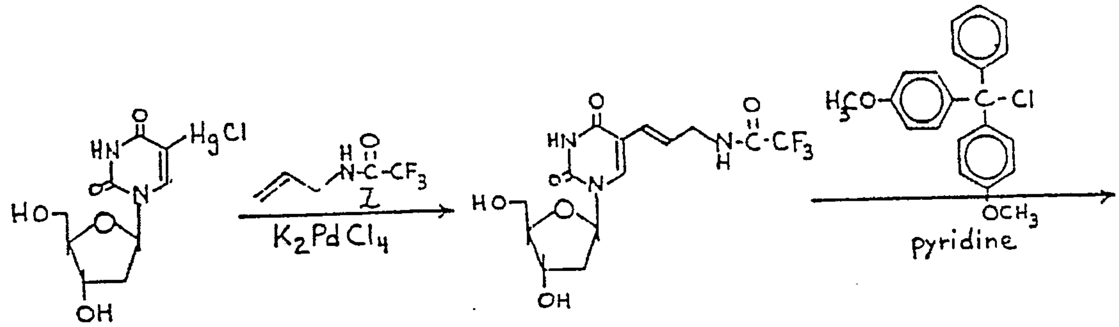


5 8

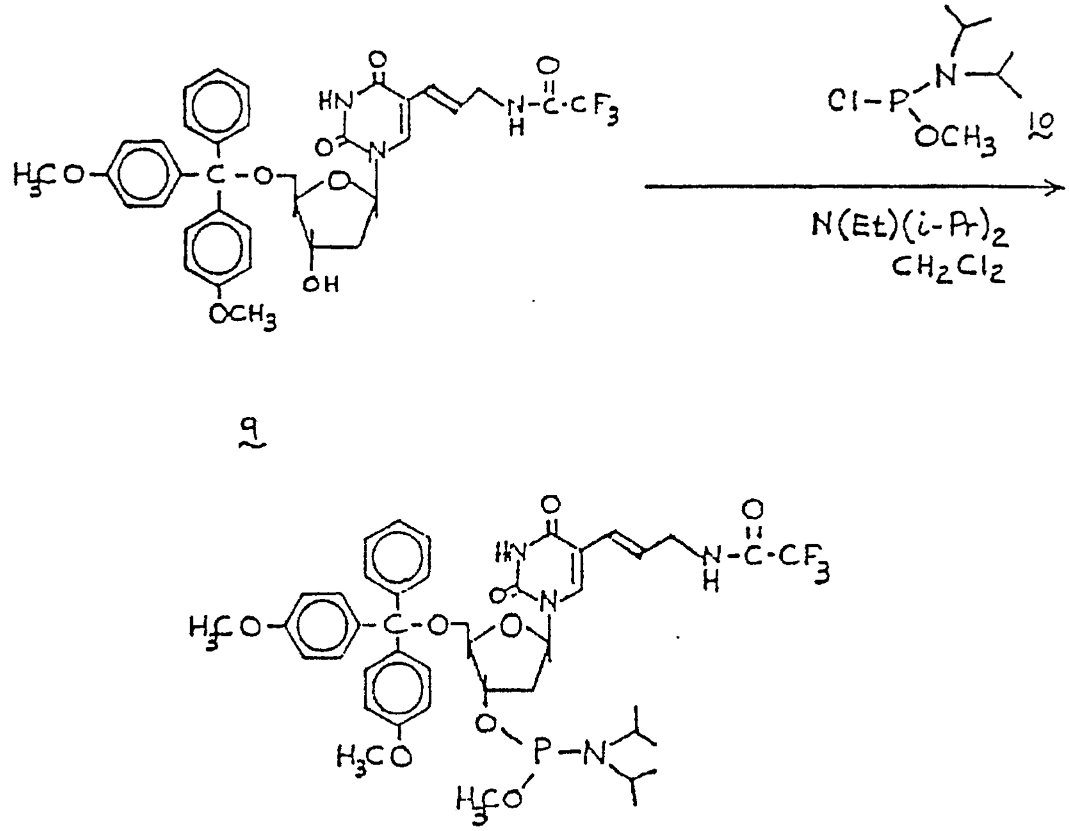


9

1

Scheme for the synthesis of 1 to be added to 19A' at the 5' end.

The synthesis of 1 is outlined in the above scheme.

5-Chloromercuri-2'-deoxyuridine (5), prepared according to the method of D.E. Bergstrom and J.L. Ruth, J. Carbohydrates, Nucleosides and Nucleotides, 4, 257 (1977), was treated with 3-trifluoroacetamido-1-propene (7) (M. Paileand W.J. Hubsch, Monatshefte furChemie, 97, 99 (1966)) and $K_2PdCl_4$ in methanol to give 5-trifluoroacetamidoallyl- 2'-deoxyuridine (8) in 22% yield after two chromatographies and a crystallization from methanol. Reaction of 8 with 4,4'-dimethoxytrityl chloride in pyridine produced 9 in 85% yield after flash chromatography (W.C. Still, M. Kahn, A. Mitra, J. Org.Chem., 43, 2923 (1978)), which was subsequently treated with N,N-diisopropylaminomethoxy chlorophosphine (L.J. McBride and M.H. Caruthers, Tet. Letters, 24, 245 (1983)) (10) to give 1 as a white solid after precipitation from pentane.

A 19-unit oligonucleotide HB19A':
3'-GA-GGA-CTC-CTC-TTC-AGA-CG-5'
was prepared using a DNA synthesizer; three separate 1 μmole batches of each oligonucleotide were made and each was attached to a solid support and fully protected by a dimethoxy trityl radical. The dimethoxytrityl protecting group was removed from the 5'-terminus and 1 was attached to the 19-unit chain without the DNA synthesizer, but using the same reagents and conditions the machine typically employs.

The product of this process, after removal from the support, is an oligonucleotide with a 5'-(3-aminoallyl-5'-(4,4'-dimethoxytrityl)-2'-deoxy- uridine unit at the C-5' end, viz.,

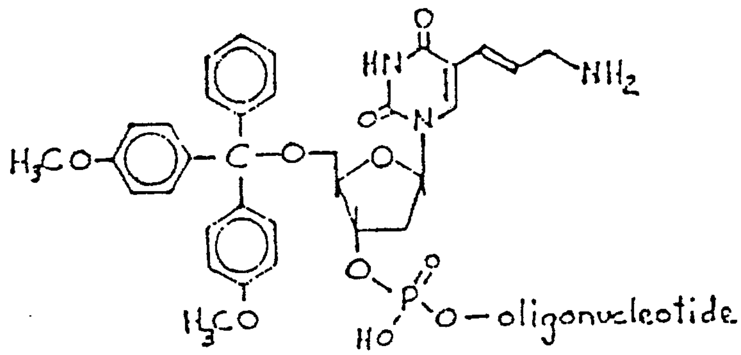

The product polynucleotides were lastly de-tritylated with brief exposure to 3% trichloracetic acid then purified by polyacrylamide gel electro- phoresis.

The process of attaching an analogue of Bolton and Hunter (A.E. Bolton and W.H. Hunter, Biochem. J., 133, 529 (1973)) reagent is as follows:

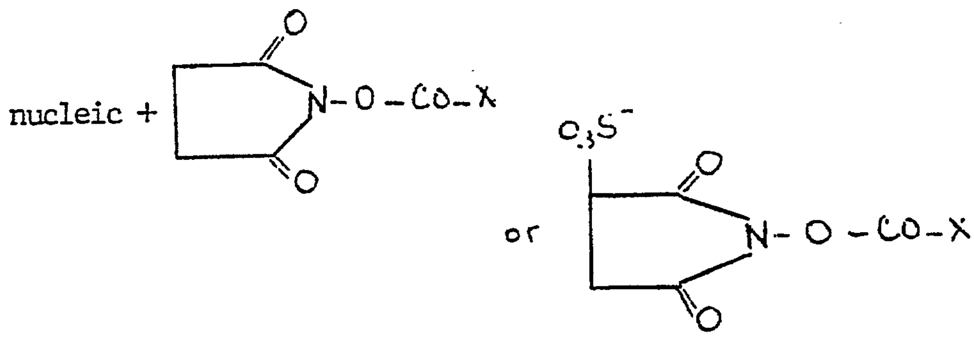
nucleic +

or

wherein X is as defined above, a specific example of the above reaction is as follows:

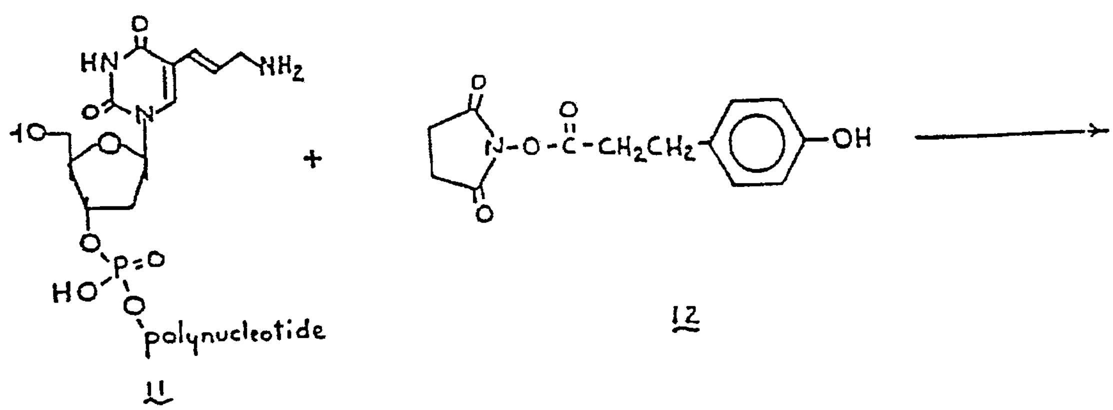
11

12

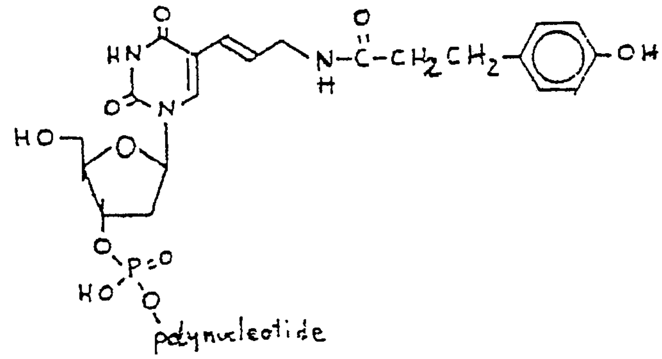

13

Iodinated in the presence of Chloramine T

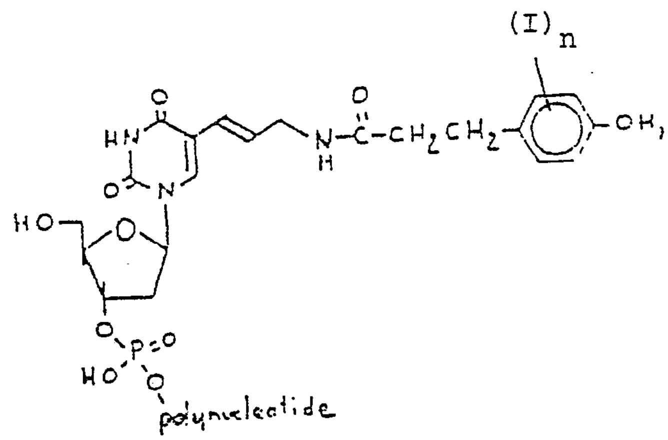

14

wherein n is 1 to 4, preferably 1 to 2, i.e., reaction of the amino-functionalized polynucleotide 11 with the N-hydroxysuccinimide ester of 3-(4'-hydroxy-phenyl) propionic acid (12) yields the probe polynucleotide 13 containing a hydroxyphenyl residue which can be iodinated in the presence of chloramine T to produce compound 14.

The polynucleotide HB19A' is a 19 unit polynucleotide corresponding to a portion of human DNA which codes for the polypeptide β hemoglobin, specifically that region of the DNA wherein lies the mutation which manifests itself in the formation of sickle-cell hemoglobin and the genetic disorder known as sickle cell anemia.

Infrared (IR) spectra were obtained as solutions in $CHCl_3$ unless otherwise noted; the 1602 $cm^{-1}$ band of polystyrene film was used as an external calibration standard.

Proton magnetic resonance ($^1H$ NMR) spectra were obtained in $CDCl_3$ solution unless otherwise noted. Chemical shifts are reported in parts per million downfield from the internal standard tetramethylsilane, unless otherwise noted.

Carbon-13 magnetic resonance ($^{13}C$ NMR) spectra were obtained in $CDCl_3$ solution unless otherwise noted. Carbon shifts are reported in parts per million downfield from the internal standard tetramethyl-silane, unless otherwise noted.

Phosphorus-31 magnetic resonance ($^{31}PNMR$) spectra were obtained in $CDCl_3$ solution unless otherwise noted. Phosphorus shifts are reported in parts per million downfield from an external aqueous 15% $H_3PO_4$ stardard.

Thin layer chromatograph (TLC) was performed using silica gel 60F-254 plates from E. Merck. Column chromatography was performed using E. Merck Silica Gel 60 (70-230 mesh).

5-Trifluoroacetamidoallyl-2'-deoxyuridine (8)

A suspension of 5-chloromercuri-2'-deoxyuridine (5) (Bergstrom and Ruth, supra) (5.56 g; 12 mmol) in HPLC (high performance liquid chromatography) grade methanol (120 ml) was maintained under an inert gas atmosphere at ambient temperature and treated, with 3-trifluoroacetamido-1-propene (7) (Pailer and Hubsch, supra) (7.33 g; 48 mmol; 4 equivalents), and $K_2PdCl_4$ (4.28 g; 1.1 equivalents). The reaction gradually becomes black and was allowed to stir for 22 hours. The mixture was treated with $H_2S$ gas for several minutes then filtered through Celite, rinsed with methanol and evaporated to dryness under reduced pressure from a 80°C bath to give a crude semi-solid residue (7.0 g). The residue was chromatographed on a silica gel column developed with $CH_2Cl_2$ : MeOH (5:1). The band which stained a blue color with modified p-anisaldehyde reagent (Egon Stahl, Thin LayerChromatography, 2nd Edition, Springer-Verlog, N.Y., 857 (1969)) and had an Rf = 0.51 ($CH_3CN$ : MeOH 3:1) was collected and evaportated to dryness in vacuo to give a colorless foam. The product was crystallized from a minimum of methanol, filtered, washed with cold $CHCl_3$ : MeOH (3:1) and vacuum dried. The mother liquor was worked for a second crop-total yield 1.01 g (22%). A recrystalization from MeOH produced the title compound (8) as analytically pure tiny white needles with mp = 183-4° after drying in vacuo (<1.0 torr) at 64°C overnight. IR (KBr) $cm^{-1}$ 3420, 3260, 1718, 1683 (br), 1560, 1478, 1283, 1190, 1102, 1061, 980, 788, 763, 737; 'HNMR (DMSO-$d^6$) (Ref. DMSO-$d^6$) δ 2.13 (d of d, J = 6 Hz, 2H), 3.59 (br s, 2H), 3.70-3.97 (m, 3H), 4.25 (br s, 1H), 5.06 (br m, 1H), 5.20 (br m, 1H), 6.05-6.65 ($m_6$, 4H), 8.01 (s, 1H), 9.60 (br s, 1H); $^{13}C$ NMR (DMSO-$d^6$) (Ref. DMSO-d)ppm 162.05, 155.29, 149.50, 138.05, 124.33, 124.14, 109.96, 87.53, 84.47, 70.23, 61.12, 39.93; $[\alpha]_D$ = +8.01° (c = 0.87, MeOH).

| | | | |
|---|---|---|---|
| Anal. Calcd. for $C_{14}H_{16}N_3O_6F_3$: | C, 44.33; | H, 4.25; | N, 11.08 |
| Found: | C, 44.19; | H, 4.10; | N, 10.93 |

5-Trifluoroacetamidoallyl-5'-0-(4,4'-dimethoxytrityl)-2'-deoxyuridine (9)

A solution of 8 (0.60 g; 1.58 mmol) in anhydrous pyridine (8 ml) was maintained under an inert gas atmosphere and treated at ambient temperature with 4,4'-dimethoxytrityl chloride (0.67 g; 1.25 equivalents). After stirring for 18 hours the reaction was poured into ice water (70 ml) with vigorous shaking. On standing 1/3 hours at 0°C, a gummy solid separated, leaving a nearly clear solution which was decanted. The solid was washed once with $H_20$ (5 ml) then taken up in $CH_2Cl_2$(10 ml), washed once with brine (5ml) then the $CH_2Cl_2$ solution was dried over $K_2CO_3$, filtered and evaporated to dryness in vacuo to give a brownish foam. The crude product was purified by flash chromatography (Still et al, supra) on a column of silica gel (Merck, Grade 60, 230-400 mesh, 60 A) (75 g) developed with 4.0% MeOH in $CHCl_3$ solvent (1.0l). Fractions of ca. 20 ml each were collected in tubes containing pyridine (10 µl) to inhibit deprotection of the 5'-hydroxyl. Fractions containing the major product band (RF = 0.29; MeOH : $CHCl_3$ 7.93) were combined, filtered and evaporated to dryness in vacuo to give 9 (0.91 g; 85%) as a slightly yellowish foam. A fraction from the center of the elution band was freed of solvent, taken up in EtoAc, treated with Norit 211, filtered through Celite and evaporated to dryness under high vacuum (<1.0 torr) at 64°C overnight to afford the analytical sample as a colorless foam with mp = 105-110°C (dec.). IR ($CHCl_3$) $cm^{-1}$ 3370, 2920, 1715, 1695, 1618, 1515, 1470, 1260, 1182, 1045, 842; H'NMR ($CDCl_3$) δ 2.38 (br m, 2H) , 3.25-3.75 (m, 5H) , 3.75 (s, 6H) , 4.10 (br m. 1H) , 4.60 (br s, 1H) , 5.39 (d, J = 16 Hz, 1H) , 6.10-6.55 (m, 2H) , 6.70-6.95 (m, 5H) , 7.15-7.45 (m, 10H) , 7.84 (s, 1H) ; $^{13}C$ NMR ($CDCl_3$) (Ref. $CDCl_3$) ppm 162.31 , 158.74 , 157.70 , 156.01 , 149.70 , 144.04 , 137.88 , 135.65 , 135.52 , 130.12 , 128.11 , 127.26 , 125.05 , 113.48 , 111.33 , 86.94 , 96.68 , 85.25 , 72.18 , 63.60 , 55.34 , 42.66 , 41.42 .

| | | | |
|---|---|---|---|
| Anal. Calcd. for $C_{35}H_{34}N_3O_8F_3$: | C, 61.67; | H, 5.03; | N, 6.16 |
| Found: | C, 61.47; | H, 5.19; | N, 5.95 |

5-Trifluoroacetamidoallyl-5'-0-(4,4'-dimethoxytrityl)-2'-deoxyuridine-3'-0-(N,N-diisopropylaminomethoxy phospine) (1)

A solution of 9 (0.34 g; 0.5 mmol) in anhydrous $CH_2Cl_2$ (1.5 ml) maintained under an Argon atmosphere at ambient temperature was treated first with anhydrous, diisopropylethylamine (0.35 ml; 0.259 g; 2mmol; 4 equivalents) then dropwise, over 1 minute with N, N-diisopropylaminomethoxy-chlorophosphine (see McBride et al, supra) (10) (0.19 ml; ca. 0.2 g; 2.2 equivalents). The resultant colorless solution is stirred for 20 minutes then transferred with EtOAc (20 ml) (EtOAc was previously washed with saturated aq $NaHCO_3$, then brine) to a separatory funnel, washed four times with brine (35 ml each), dried over $Na_2SO_4$, filtered and evaporated to dryness in vacuo to give a colorless glass (0.51 g). This crude product was taken up in anhydrous benzene (2 ml) and precipitated into rapidly stirred anhydrous pentane (60 ml) at -78°C under an argon atmosphere. The resulting suspension was filtered, washed with -78°C pentane and vacuum dried at <1 torr over KOH overnight to obtain the title compound (1) (0.38 g; 93%) as a

white amorphous powder. IR ($CHCl_3$) $cm^{-1}$ 2965, 1722, 1698, 1618, 1518, 1470, 1262, 1185, 1045, 988, 842; 'H NMR ($CD_2Cl_2$) δ 0.95-1.30 (m, 12H), 2.20-2.60 (m, 2H), 3.24 and 3.37 (d of d, J = 13 Hz, 3H) (P-O-$CH_3$), 3.20-3.80 (m, 6H), 3.75 (s, 6H), 4.17 (br m, 1H), 4.68 (v br m, 1H), 5.42 (d, J = 16 Hz, 1 H), 6.15-6.55 (m, 3H), 6.75-6.95 (m, 4H), 7.20-7.50 (m, 10H, 7.79 (s, 1H); $^{13}C$ NMR ($CD_2Cl_2$) (Ref. $CD_2Cl_2$) ppm 162.40, 159.21, 157.78, 149.78, 144.71, 138.34, 136.00, 130.53, 128.71, 128.45, 127.54, 125.66, 125.27, 113.82, 111.48, 87.23, 86.31, 85.60, 55.75, 43.78, 43.20, 42.94, 24.99, 24.60; $^{31}P$NMR ($CD_2Cl_2$) ppm 149.30, 148.87, 14.11 (ca. 12% impurity), 8.18 (ca. 4% impurity).

Attachment of 1 to Oligonucleotides

The 19-unit oligonucleotides were synthesized using an Applied Bio-systems Model 380A DNA Synthesizer on control pore glass solid support. Immediately prior to attaching 1 to the 5' end of the oligomer, the 5'-0-(4,4'-dimethoxytrityl) protecting group was cleaved on the machine with 3% $CCl_3CO_2H$ in $CH_2Cl_2$ for 90 seconds. The support-bound 5'-deprotected oligomer was washed with $CH_3CH$ and dried in an argon stream. Subsequent steps were performed without the machine, but using the same chemistry;

1. The support-bound oligomer was removed from the container (column) used for automated synthesis and transferred to a dry septum-cap vial under an Argon atmosphere
2. The bound oligomer was treated with a 20-30 fold excess of 0.5 M 1H-tetrazole in anhydrous $CH_3CN$ followed immediately with a similar excess of 1 in $CH_3CN$. It was incubated 30 minutes with gentle agitation.
3. Reagents were pipetted off and the bound oligomer was washed with three portions of $CH_3CN$.
4. The solid support containing the bound oligomer was treated with an excess of $I_2$-$H_2O$-Lutidine-THF (0.1 M : 1:10:40) and agitated for 15 minutes.
5. Reagents were pipetted and the bound oligomer was washed with four portions of $CH_3CN$.
6. The solid support containing the bound oligomer was washed with an excess of thiophenol-triethylamine-dioxane for 60 minutes.
7. Reagents were pipetted off and the bound oligomer was washed with four portions of MeOH.
8. The solid support containing the bound oligomer was treated with concentrated aqueous $NH_4OH$ for 2 hours at ambient temperature (this removes protected oligonucleotide from the support).
9. To remove all protecting groups the oligonucleotide was treated with concentrated aqueous $NH_4OH$ and heated at 50°C overnight (this removes all protecting groups except the dimethoxytrityl).
10. The support was filtered off and the filtrate was evaporated to dryness to obtain crude oligonucleotide.

The above ten steps were repeated for all batches of support-bound oligonucleotide. Treatment of a portion of each on a silica gel TLC plate with 3% $CCl_3CO_2H$ in $CH_2Cl_2$ produced the orange-red color of dimethoxytrityl cation indicating the successful incorporation of 1 into the oligonucleotides.

One batch of the modified HB19A' oligonucleotide was detritylated with 3% $CCl_3CO_2H$ in $CH_2Cl_2$, then purified by electrophoresis on polyacrylamide gel.

Example 2

The reaction of a specific oligonucleotide with sulfosuccinimidyl hydroxy phenyl propionate:

Two micrograms of 19A' amine from Example 1 were dissolved in 20 microliters of 10 mM borate buffer pH 8.16. To this, 5 microliters of a freshly prepared solution of sulfosuccinimyl 3-(4-hydroxyphenyl)-propionate (SHPP) purchased from Pierce were added. The concentration of SHPP was 5 mg per ml in water. The reaction was allowed to proceed at room temperature for 30 minutes then it was kept in a freezer before HPLC separation was conducted. After the reaction, the extent of the reaction was monitored by running a 20% polyacrylamide gel in conventional manner.

Example 3

The separation of SHPP reacted 19A' from the reaction mixture containing hydrolyzed SHPP and oligonucleotides:

HPLC separation was conducted on a Brownlee RP300 guard column coupled to a Synchropack RPP 4.1 x 10 cm (Synchrom; Linden, Indiana) column at ambient temperature with a gradient of .1 M triethylammonium acetate pH 7 to 0.1 M triethylammonium acetate pH 7, 50% acetonitrile was run over a period of 10 - 120 minutes depending on the sample. The detector was set at 254 nm and full scale was 0.15 absorbance unit. In order to determine the location of the derivatized oligonucleotide product, a blank run had been carried out with the reaction mixture without the oligonucleotide. A new peak appeared after adding the oligonucleotide and corresponded to the reaction product. After the product was separated and collected in a fraction collector, the product was analyzed by gel electrophoresis and from the next run an analytical determination of the proper peak was not deemed necessary. The yield and recovery of the product from the columns was between 80% to 90%.

Example 4

Iodination of SHPP labeled oligonucleotide:

The HPLC fraction containing the SHPP labeled oligonucleotide was evaporated under reduced pressure and then was redissolved in 5 microliters of water. The 5 microliter aqueous solution containing 2 microgram of the SHPP labeled oligonucleotide was diluted to 50 microliter with phosphate buffer pH 7.1. The buffer contained 100 mmoles disodium hydrogen phosphate and 50 mmoles sodium dihydrogen phosphate. Then one microliter of 0.1 normal hydrochloric acid was added to this 55 microliter solution followed by one microliter of $^{125}I$, New England Nuclear, 400 microcuries and one Iodobead (immobilized chloramiue T) from Pierce are added. The reaction was continued for 15 minutes in ice water. The reaction was terminated by removing the Iodobead, and injection of the solution onto a reverse-phase chromatographic system.

Example 5

High pressure liquid chromotographic separation of the iodinated oligonucleotide from the non-iodinated oligonucleotide and sodium iodide:

The mixture containing the oligonucleotide and the iodination reagents was diluted with triethylammonium acetate buffer then they are loaded onto a guard column (Example 3). First the column was washed with 0.1 molar triethylammonium acetate to remove sodium iodide and any other components which were not absorbed onto the reverse-phase column. After the first large radioactive peak (which did not contain any oligonucleotide) eluted, the guard column was put in series with an analytical column (Sychropack RP-P) and the gradient of 0 to 50% acetonitrile in 0.1 M triethylammonium acetate was run through the system. In a typical run the uniodinated material eluted prior to the iodinated SHPP labeled oligonucleotide. After the peaks were collected they were again analyzed on gel electrophoresis. This analysis was not necessary after establishing the chromatographic properties.

Example 6

Hybridization of this 19A' allylamino UTP labeled oligonucleotide labeled with iodine:

The specific oligonucleotide fragment chosen for this example was usable for the detection of sickle cell anemia. This particular sequence of oligonucleotide 5'-U* -GCA GAC TTC TCC TCA GGA G - 3' (U* = 5 allyamino U) will hybridize with DNA extracted from normal blood, whereas under stringent conditions it will not hybridize to the DNA extracted from the blood of a person who has sickle cell anemia disease. The extraction of DNA from blood samples and the digestion with restriction enzymes and other treatment of the hybridization processes have been described in detail in a paper by Conner, B.J. et a., <u>supra</u>, with similar oligonucleotide lacking U*.

The material iodinated by the present method can be used for the detection of sickle cell anemia.

It will be appreciated that the instant specification and claims are set forth by way of illustration and not limitation, and that various modifications and changes may be made without departure from the spirit and scope of the present invention.

## Claims

1. A process for producing an iodinated probe of the formula

$$\text{Nucleic Acid-R-NH-CO-}\overset{(I)_n}{\underset{}{X}}$$

wherein X is an iodinatable residue, R is an aliphatic hydrocarbon radical of up to 11 carbon atoms and n = 1 to 4, which process comprises reacting a nucleic acid having a nucleotide residue containing a primary amino radical with a compound having the formula

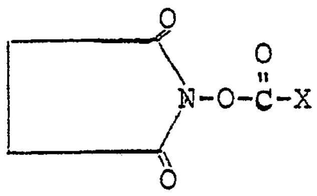

or

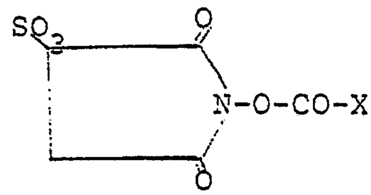

wherein

X is as defined above and iodinating the product with an iodinating agent.

2. The process according to claim 1 wherein the iodinatable residue is selected from the group comprising hydroxyaryl, preferable hydroxyphenyl radicals such as

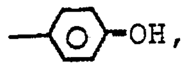

tryptophane, indole, histidine or methionine, cysteine and cystine.

3. The process according to any of claims 1 and 2 wherein X is

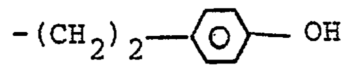

4. The process according to any of claims 1 to 3 wherein R is attached to the sugar of the nucleic acid or to the heterocyclic moiety of the nucleic acid.

5. The process according to any of claims 1 to 4 wherein R has 3 to 6 carbon atoms.

6. The process according to any of claims 1 to 5 wherein the amino radical in the amine containing nucleotide is a primary aliphatic amine residue.

7. The process according to any of claims 1 to 6 wherein the amine containing nucleotide residue 5-allylaminodU, or 8- or 6-hexylaminodA or is A, or a 5-allylamino C or C or an oxidatively coupled amino containing A, G, T, C, U.

8. Use of the iodinated probe prepared by the process of any of claims 1 to 7 in nucleic acid hybridization.

**Patentansprüche**

1. Verfahren zur Herstellung einer iodierten Probe der Formel

$$\text{Nucleinsäure-R-NH-CO-}\overset{(I)_n}{\overset{|}{X}}$$

worin X ein iodierbarer Rest, R ein aliphatischer Kohlenwasserstoffrest von bis zu 11 Kohlenstoffatomen und n = 1 bis 4 sind, wobei man eine Nucleinsäure mit einem Nucleotidrest, der einen primären Aminorest enthält, mit einer Verbindung mit der Formel

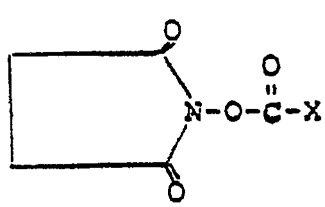

oder

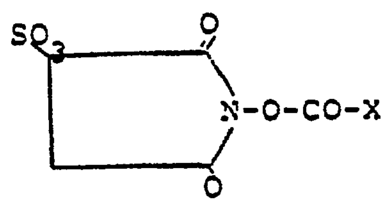

umsetzt, worin X wie vorstehend definiert ist, und das Produkt mit einem Iodierungsmittel iodiert.

2. Verfahren gemäß Anspruch 1, dadurch gekennzeichnet, daß der iodierbare Rest aus der Gruppe ausgewählt ist, umfassend Hydroxyaryl, vorzugsweise Hydroxyphenyl

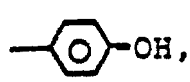

Tryptophan, Indol, Histidin oder Methionin, Cystein und Cystin.

3. Verfahren gemäß jedem der Ansprüche 1 und 2, dadurch gekennzeichnet, daß X

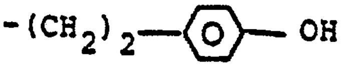

ist.

4. Verfahren gemäß jedem der Ansprüche 1 bis 3, dadurch gekennzeichnet, daß R an den Zucker der Nucleinsäure oder an den Heterocyclus der Nucleinsäure gebunden ist.

5. Verfahren gemäß jedem der Ansprüche 1 bis 4, dadurch gekennzeichnet, daß R 3 bis 6 Kohlenstoffatome besitzt.

6. Verfahren gemäß jedem der Ansprüche 1 bis 5, dadurch gekennzeichnet, daß der Aminrest im Amin enthaltenden Nucleotid ein primärer aliphatischer Aminrest ist.

7. Verfahren gemäß jedem der Ansprüche 1 bis 6, dadurch gekennzeichnet, daß der Amin enthaltende Nucleotidrest 5-AllylaminodU oder 8- oder 6-HexylaminodA oder -A oder ein 5-AllylaminodC oder -C oder ein oxidativ gekuppeltes Amino enthaltendes A, G, T, C, U ist.

8. Verwendung der iodierten Sonden, hergestellt durch das Verfahren gemäß eines jeden Anspruchs 1 bis 7, in Nucleinsäurehybridisierungen.

**Revendications**

1. Procédé de production d'une sonde iodée de formule

$$\text{acide nucléique-R-NH-CO-}\overset{(I)_n}{\underset{}{X}}$$

dans laquelle X est un résidu iodable, R est un radical hydrocarbure aliphathique ayant jusqu'à 11 atomes de carbone et n = 1 à 4, ce procédé comprenant la réaction d'un acide nucléique ayant un résidu nucléotide contenant un radical amino primaire avec un composé de formule

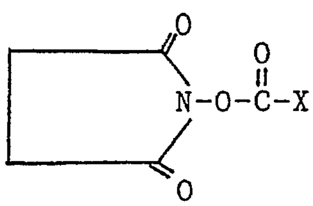

ou

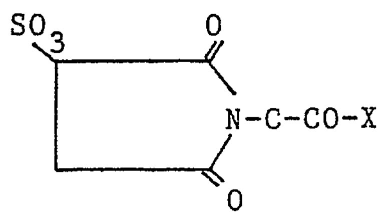

dans laquelle X est comme défini ci-dessus, et l'iodation du produit avec un agent iodant.

2. Procédé selon la revendication 1, dans lequel le résidu iodable est choisi parmi le groupe comprenant hydroxyaryle, de préférence hydroxyphényle, des radicaux tels que

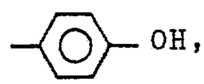

tryptophane, indole, histidine ou méthionine, cystéine et cystine.

3. Procédé selon n'importe laquelle des revendications 1 et 2, dans lequel X est

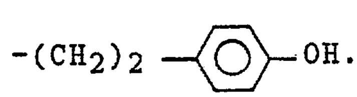

4. Procédé selon n'importe laquelle des revendications 1 à 3, dans lequel R est fixé au sucre de l'acide nucléique ou à la fraction hétérocyclique de l'acide nucléique.

5. Procédé selon n'importe laquelle des revendications 1 à 4, dans lequel R a 3 à 6 atomes de carbone.

6. Procédé selon n'importe laquelle des revendications 1 à 5, dans lequel le radical amino dans le nucléotide contenant l'amine est un résidu d'amine aliphatique primaire.

7. Procédé selon n'importe laquelle des revendications 1 à 6, dans lequel le résidu nucléotide contenant l'amine est 5-allylaminodU, ou 8- ou 6-hexylaminodA ou est A, ou un 5-allylamino C ou C ou un amino couplé par oxydation contenant A, G, T, C, U.

8. Emploi de la sonde iodée préparée par le procédé selon n'importe laquelle des revendications 1 à 7 dans une hybridation d'acide nucléique.